(19) **Europäisches Patentamt / European Patent Office / Office européen des brevets**

(11) **EP 4 306 122 A1**

(12) **EUROPEAN PATENT APPLICATION**
published in accordance with Art. 153(4) EPC

(43) Date of publication:
**17.01.2024 Bulletin 2024/03**

(21) Application number: **22766373.9**

(22) Date of filing: **11.03.2022**

(51) International Patent Classification (IPC):
**A61K 36/605** (2006.01)    **A61P 3/04** (2006.01)
**A61P 3/06** (2006.01)    **A61P 3/10** (2006.01)

(86) International application number:
**PCT/CN2022/080278**

(87) International publication number:
**WO 2022/188851 (15.09.2022 Gazette 2022/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.03.2021 CN 202110272018**
**19.11.2021 CN 202111400867**
**02.12.2021 CN 202111460847**

(71) Applicants:
- **Beijing Wehand-Bio Pharmaceutical Co., Ltd**
  **Beijing 102600 (CN)**
- **Guangxi Wehand-Bio Pharmaceutical Co., Ltd**
  **Hechi, Guangxi 546300 (CN)**

(72) Inventors:
- **LIU, Yuling**
  **Beijing 102600 (CN)**
- **CHEN, Yanmin**
  **Beijing 102600 (CN)**
- **LIU, Zhihua**
  **Beijing 102600 (CN)**
- **ZHU, Xiangyang**
  **Beijing 102600 (CN)**
- **JIN, Yiqun**
  **Beijing 102600 (CN)**
- **YANG, Hongzhen**
  **Beijing 102600 (CN)**
- **WANG, Tingting**
  **Beijing 102600 (CN)**
- **LIU, Yuanyuan**
  **Beijing 102600 (CN)**
- **SHEN, Zhufang**
  **Beijing 102600 (CN)**
- **LIU, Shuainan**
  **Beijing 102600 (CN)**
- **LIU, Quan**
  **Beijing 102600 (CN)**
- **LIAN, Chunfang**
  **Beijing 102600 (CN)**
- **SUN, Qianwen**
  **Beijing 102600 (CN)**
- **ZOU, Yuanyuan**
  **Beijing 102600 (CN)**
- **LI, Caina**
  **Beijing 102600 (CN)**
- **LEI, Lei**
  **Beijing 102600 (CN)**
- **CAO, Hui**
  **Beijing 102600 (CN)**

(74) Representative: **Bandpay & Greuter**
**30, rue Notre-Dame des Victoires**
**75002 Paris (FR)**

(54) **USE OF MULBERRY EXTRACT IN PREPARATION OF DRUG FOR REDUCING WEIGHT OF ANIMAL**

(57)    The present disclosure relates to a mulberry extract used in preparation of a drug for reducing animal weight. The mulberry extract comprises: 3% to 99% of an alkaloid, 0.2% to 70% of a polysaccharide, 0% to 50% of an amino acid, 0% to 10% of flavone, and 0% to 25% of other components. The mulberry extract of the present disclosure can be used for controlling weight gain, and has a good therapeutic effect on type 2 diabetes with obesity.

Fig. 1

## Description

### Technical Field

The present disclosure relates to use of a mulberry extract in preparation of a drug for reducing animal weight.

### Background Art

As our life improves, obesity and overweight are increasing at an alarming rate all over the world. Epidemiological studies have shown that obesity and overweight are major risk factors for diabetes, cardiovascular diseases, cancers and premature death. The causes of obesity include increases in adipocyte size and number, wherein the increase in adipocyte number is caused by the differentiation of preadipocytes into mature adipocytes, and the differentiation of preadipocytes into mature adipocytes is accomplished by the activation and expression of a series of transcription factors. Data show that the number of diabetics is the largest in the world, of which the proportion of overweight people is 41.0% and the proportion of obese people is 24.3%. Relative to patients with simple obesity, it is more difficult for type 2 diabetics with obesity to lose and maintain weight (Experts' Consensus in China on Integrated Management of Type 2 Diabetes with Obesity, China J Endocrinol Metab, 2016, 32(08): 623-627).

Due to their high nutritional value and medicinal value, Moraceae plants have been considered as precious materials for both medicine and food ever since ancient times. There are records in traditional Chinese medicine classics and prescriptions of all ages that Ramulus Mori, Cortex Mori, Folium Mori and mulberries were used for treatment of diseases. For example, as early as in Compendium of Materia Medica, there are descriptions of "the decoction of the Folium Mori juice can quench thirst in place of tea" and "drinking after being cooked and decocted can quench thirst in place of tea". Chemical components in Moraceae plants mainly include flavone compounds, polysaccharide compounds, alkaloids or the like, and now are widely used in the preparation of hypoglycemic, lipid-lowering, antiviral and immunomodulatory drugs.

In prior art, there are some reports about mulberry extract's effect on reducing weight and lowering lipid. For instance, CN1631246A, an application for patent, discloses the weight reducing and lipid lowering effects of Ramulus Mori extract, which is specifically shown as that ethanol extract of Ramulus Mori can reduce weight of NIH mice, as well as lower blood triglyceride and cholesterol levels, but neither the administration method nor the dose-effect relationship thereof is clear; CN102370708A, another application for patent, discloses use of water extract or alcohol extract of Chinese herbal medicine Folium Mori in preparation of weight-losing and lipid-lowering medicines, and discloses the use of mulberry extract for reducing weight and lowering lipid.

KKAy mice are a kind of animal model for type 2 diabetes and are formed by transference of mutant gene (ay) into KK mice; the cause thereof is environmental factor inducing on the basis of genetic susceptibility, and is very similar to the performance of human type 2 diabetes; moreover, with small individual differences and good experimental repeatability, they are a relatively ideal animal model of spontaneous type 2 diabetes. KKAy mice have clinical features of polydipsia, polyphagia and urorrhagia with obesity, and are characterized by hyperglycemia, high insulin resistance, islet insufficiency, liver diseases, kidney diseases and so on. Studies show that the body weight, and liver fat and epididymal fat weight of KKAy mice are significantly higher than that of normal mice. At present, there is no research report on the effect of mulberry related extract with clear composition on the weight of KKAy mice.

### Summary of the Invention

It is studied and discovered by the inventor that the mulberry extract in the present disclosure has a significant effect on reducing animal weight. On such a basis, the present disclosure provides use of a mulberry extract in preparation of a drug for reducing animal weight.

In one embodiment of the present disclosure, the mulberry extract comprises an alkaloid, a polysaccharide, an amino acid and flavone. Preferably, on the basis of the mulberry extract, weight contents of each component are as follows:

| | |
|---|---|
| an alkaloid | 3% to 99%, |
| a polysaccharide | 0.2% to 70%, |
| flavone | 0% to 10%, |
| an amino acid | 0% to 50%, and |
| other components | 0% to 25%; |

more preferably, on the basis of the mulberry extract, weight contents of each component are as follows:

| an alkaloid | 50% to 99%, |
| a polysaccharide | 0.2% to 35%, |
| flavone | 0% to 2%, |
| an amino acid | 0% to 30%, and |
| other components | 0% to 20%; and |

further preferably, on the basis of the mulberry extract, weight contents of each component are as follows:

| an alkaloid | 50% to 99%, |
| a polysaccharide | 0.2% to 25%, |
| flavone | 0% to 1%, |
| an amino acid | 0% to 20%, and |
| other components | 0% to 20%. |

In one embodiment, preparation of the mulberry extract comprises following steps of: preparing a crude extraction solution; optionally, separating by means of cation resin and/or anion resin; optionally, subjecting resin effluent to alcohol precipitation; and, optionally, concentrating and drying. Preferably, preparation of the mulberry extract comprises following steps of: step 1): preparing a crude extraction solution; step 2): separating by means of cation resin and/or optional anion resin; an optional step 3): subjecting resin effluent obtained in step 2) to alcohol precipitation; and an optional step 4): concentrating and drying.

In one embodiment, the mulberry extract was prepared according to following steps of: crushing Ramulus Mori, Folium Mori or Cortex Mori; heating for reflux extraction with water and/or alcohol solution or acid water, solvent amount being 3 to 20 times as that of original medicine materials; repeating the extraction for 1 to 3 times; combining extraction solution and concentrating; loading cation exchange resin; washing away non-adsorbed impurities with distilled water and eluting with 0.2 to 3 N ammonium hydroxide; concentrating with an eluent and loading anion exchange resin; collecting non-adsorbed part, adding ethanol, precipitating to remove impurities, centrifuging, and subjecting to concentration under reduced pressure by means of a clear solution or spray drying or freeze drying, thereby obtaining extract.

In one embodiment, the mulberry extract was prepared according to the following steps of: crushing Ramulus Mori, Folium Mori or Cortex Mori; heating for reflux extraction with water and/or alcohol solution or acid water, solvent amount being 3 to 20 times as that of original medicine materials; repeating the extraction for 1 to 3 times; combining extraction solution and concentrating; loading cation exchange resin; washing away non-adsorbed impurities with distilled water and eluting with 0.2 to 3 N ammonium hydroxide; concentrating with an eluent and loading anion exchange resin; collecting non-adsorbed part, and subjecting to concentration under reduced pressure or spray drying or freeze drying, thereby obtaining extract.

In one embodiment, the mulberry extract was prepared according to following steps of: crushing Ramulus Mori, Folium Mori or Cortex Mori; heating for reflux extraction with water and/or alcohol solution or acid water, solvent amount being 3 to 20 times as that of original medicine materials; repeating the extraction for 1 to 3 times; combining extraction solution and concentrating; loading cation exchange resin; washing away non-adsorbed impurities with distilled water and eluting with 0.2 to 3 N ammonium hydroxide; and subjecting to concentration under reduced pressure by means of an eluent or spray drying or freeze drying, thereby obtaining extract.

The animal kind referred to by the term "animal" in the present disclosure is not specifically limited. The animals can be any animal with intrialinal organs, preferably mammals, more preferably rats, mice and humans, and most preferably humans.

In one embodiment of the present disclosure, the animals are diabetic. Preferably, the diabetic animals have a body mass indicator of BMI≥28.

Preferably, the animals are healthy.

In one embodiment of the present disclosure, the drug reduces animal weight by reducing fat accumulation in cells. Preferably, the cells are liver cells and/or epididymal adipocytes. More preferably, the cells are liver cells.

Preferably, the drug further comprises a pharmaceutically acceptable carrier. The carrier may be an inactive component that conforms to administration routes or modes and is non-toxic to human body. The carrier may be a solid or liquid excipient. Solid excipients, for example, include microcrystalline cellulose, mannitol, lactose, pre-gelled starch, low-substituted hydroxypropyl cellulose, polyvinylpolypyrrolidone, sodium carboxymethyl starch, aspartame, calcium hydrogen phosphate, sodium lactate, poloxamer, sodium dodecyl sulfate, sodium carboxymethyl cellulose, gelatin, xanthan gum, povidone, starch, magnesium stearate, sodium carboxymethyl starch and talc; and liquid excipients, for example, include water, ethanol, syrup and glycerin.

Preferably, the drug has a dosage form of oral administration; further preferably, the drug is a tablet, a capsule, an oral solution, an oral emulsion, a pill, a granule, syrup and powder.

The mulberry extract of the present disclosure can control the weight gain of mice in an obesity model of spontaneous diabetes; moreover, with clear ingredients, controllable quality, zero adverse reaction, high safety and low cost, it provides a new choice for diabetic obese people, as well as a new variety for developing and screening weight-reducing drugs.

## Brief Description of Drawings

Fig. 1 shows effects of long-term administration of the mulberry extract in Embodiment 9 on the weight of KKAy mice: (A) weight of each group before and after administration, and (B) weight changes before and after administration. **$P<0.01$, ***$P<0.001$, relative to DM group.

Fig. 2 shows individual weight changes after 8 weeks of clinical administration in Embodiment 11.

Fig. 3 shows individual weight changes after 24 weeks of clinical administration in Embodiment 11.

Fig. 4 shows weight changes of high-fat fed C57 mice in Embodiment 13 after intragastric administration.

Fig. 5 shows weight changes of high-fat fed C57 mice in Embodiment 13 after injection administration.

Fig. 6 shows the effects of different doses of SZ-A on liver triglyceride and cholesterol accumulation caused by palmitic acid. SZ-A stands for SZ-A treatment group of Ramulus Mori extract, MET stands for a positive control group of metformin, PA stands for a model group of insulin resistance stimulated by palmitic acid, BSA stands for a normal control group of HepG2 cells (treated with BSA of 0.25%). **$P<0.01$, relative to the normal control group.

## Embodiments

The present disclosure will be further described in details with reference to the drawings and embodiments. Through these exemplary descriptions, the features and advantages of the present disclosure will become clearer and more definite.

The specific term "exemplary" here means "being used as an example or an embodiment, or being explanatory". Any embodiment described here as "exemplary" is not necessarily construed as being superior to or better than others.

In addition, the technical features involved in different embodiments of the present disclosure described below can be combined with one another as long as they are not in conflict.

The component contents involved in the present disclosure were detected according to known methods (see the methods disclosed in patents with publication numbers of CN111077247A and CN110393738A).

## Embodiment 1 Preparation 1 of mulberry extract

1000 kg of fresh Ramulus Mori (Morus serrata Roxb-Yuesang No. 11) was taken and crushed, then was added with 4000 L of water and was extracted by heating reflux for 2 h; extraction solution was combined, and was filtered to remove insoluble substances, and a crude extraction solution is thus obtained. The crude extraction solution was concentrated by heating until a percentage of solid substances was up to 4%, and was kept at 50°C and served as a loading solution for a cation resin column.

150 kg of D113-type macroporous and weakly acidic phenylpropene-based cation resin was used for column packing, and a hydrochloric acid solution of 2 mol/L was used for washing until the pH of an eluent was 4.5; a sodium hydroxide solution of 1 mol/L was used for washing until the pH of an eluent was 8.5; a hydrochloric acid solution of 2 mol/L was used for washing until the pH of an eluent was 4.5; and deionized water, of which the volume was 5 times as that of the column, was used for rinsing, and activation was thus completed. The concentrated extraction solution was loaded, and then was eluted with 1000 L of 2.5 mol/L ammonia water at an elution speed of 6 BV/h; the eluent was collected when the effluent from the cation column was detected to be pH>7; collection was stopped when the collection solution was up to 900 L, and the collection solution was purified directly through the anion column.

62.5 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected cation resin eluent was loaded onto the anion resin, and the effluent was collected until the effluent was up to 870 L.

The collection solution was centrifuged to remove impurities, and then was concentrated by means of a reverse osmosis membrane; a specific gravity of concentrated liquid was 1.25; the concentrated liquid was transferred to an alcohol precipitation tank, and 25 L of anhydrous ethanol was added when the stirring paddle was at 500 rpm. After finishing adding the ethanol, stirring was stopped, and alcohol precipitation was performed for 24 h; the supernatant was taken and concentrated under reduced pressure to obtain extractum of extract.

The effluent was concentrated under reduced pressure so as to obtain an extractum of Ramulus Mori extract, wherein alkaloid had a content of 52%, polysaccharide had a content of 22%, flavone had a content of 0.8%, and amino acid had a content of 20%.

**Embodiment 2 Preparation 2 of mulberry extract**

10 kg of fresh Ramulus Mori (Sangteyou No. 2) were taken and crushed, then were added with 150 L of water in 2 times, and were extracted by means of decocting for 3 h each time; extraction solution was combined, and was filtered to remove insoluble substances. The extraction solution was concentrated by heating until a percentage of solid substances was up to 8%, then was transferred to an alcohol precipitation tank, and was added with 2367.9 g (3 L) of anhydrous ethanol when the stirring paddle was at 300 rpm. After finishing adding the ethanol, stirring was stopped, and alcohol precipitation was performed for 24 h; the supernatant was taken as a loading solution for the cation resin column. 5 kg of 002SC-type strongly acidic styrene-based cation resin was used for column packing, and the cation resin was activated according to the method described in Embodiment 1. The extraction solution was loaded after concentration and alcohol precipitation, and then was eluted with 100 L of 5 mol/L potassium chloride at an elution speed of 5 BV/h; the effluent was detected with 20% silicotungstic acid, and collection was started upon generation of white precipitate; the collection was stopped until the collection solution was up to 25 L; and the collection solution was purified directly through the anion resin column.

10 kg of 711-type strongly alkaline styrene-based anion resin was used for column packing, and the anion resin was activated according to the method described in Embodiment 3. The collected cation resin eluent was loaded onto the anion resin, and the effluent was collected until the effluent was up to 15 L. The collection solution was reloaded onto the cation resin, and was separated twice by using the cation resin and the anion resin in sequence according to the afore-mentioned method.

The collection solution obtained after three column separations was centrifuged to remove impurities, and then was concentrated through a reverse osmosis membrane, wherein the specific gravity of the concentrated liquid was 1.25; it was transferred to an alcohol precipitation tank, and was added with 125 g of anhydrous ethanol when the stirring paddle was at 1000 rpm. After finishing adding the ethanol, the stirring was stopped, and alcohol precipitation was performed for 24 h; the supernatant was taken and concentrated under reduced pressure to obtain extractum of extract. In addition, fresh Cortex Mori and Folium Mori (Sangteyou No. 2) were taken again and were extracted, wherein the extraction method and parameters were the same as those described above.

In the obtained extract of Ramulus Mori, alkaloid had a content of 98%, polysaccharide had a content of 0.2%, flavone had a content of 0.05%, and amino acid had a content of 0.

In the obtained extract of Cortex Mori, alkaloid had a content of 95%, polysaccharide had a content of 2%, flavone had a content of 0.1%, and amino acid had a content of 1%.

In the obtained extract of Folium Mori, alkaloid had a content of 90%, polysaccharide had a content of 4%, flavone had a content of 0.1%, and amino acid had a content of 3%.

**Embodiment 3 Preparation 3 of mulberry extract**

1000 kg of fresh Ramulus Mori (Morus atropurpurea Roxb) was taken and crushed, then was added with 11500 L of water and was extracted by heating reflux for 2 h; extraction solution was combined, and was filtered to remove insoluble substances, and a crude extraction solution is thus obtained. The crude extraction solution was centrifuged to remove impurities, and then was concentrated by means of a reverse osmosis membrane until a percentage of solid substances was up to 1%, and served as a loading solution for a cation resin column.

300 kg of D001-type macroporous and strongly acidic styrene-based cation resin was used for column packing, and the cation resin was activated according to the method described in Embodiment 1. The crude extraction solution was loaded after concentration, and then was eluted with 5000 L of 0.04 mol/L ammonium nitrate at an elution speed of 5 BV/h; the effluent was detected with 20% silicotungstic acid, and collection was started upon generation of white precipitate; the collection was stopped until the collection solution was up to 1000 L.

The collection solution obtained after separation of cation column was concentrated by nanofiltration, and then was concentrated under reduced pressure to obtain extractum of extract.

In the obtained extract of Ramulus Mori, alkaloid had a content of 15%, polysaccharide had a content of 20%, flavone had a content of 7%, and amino acid had a content of 45%.

**Embodiment 4 Preparation 4 of mulberry extract**

333 kg of dried Ramulus Mori (Yuesang No. 11) was taken and crushed, then was added with 4000 L of water and was extracted twice by heating reflux, 1 h for each reflux; extraction solution was combined and filtered, and was concentrated until the crude drug quantity reached 1 kg/L.

150 kg of D113-type macroporous and weakly acidic phenylpropene-based cation resin was used for column packing, and a hydrochloric acid solution of 2 mol/L was used for washing until the pH of an eluent was 4.5; a sodium hydroxide solution of 1 mol/L was used for washing until the pH of an eluent was 8.5; a hydrochloric acid solution of 2 mol/L was used for washing until the pH of an eluent was 4.5; and deionized water, of which the volume was 5 times as that of the column, was used for rinsing, and activation was thus completed. The concentrated extraction solution was loaded, and then was eluted with 1000 L of 2.5 mol/L ammonia water at an elution speed of 6 BV/h; the eluent was collected when the effluent from the cation column was detected to be pH>7; collection was stopped when the collection solution was up to 900 L, and the collection solution was purified directly through the anion column.

125 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected cation resin eluent was loaded onto the anion resin, and the effluent, of which pH was greater than 8, was collected until the effluent was up to 870 L.

The collection solution obtained after anion column separation was filtered by means of micro-filtration membrane to remove impurities, and then was concentrated through a reverse osmosis membrane, wherein the specific gravity of the concentrated liquid was 1.1; it was transferred to an alcohol precipitation tank, and was added with 15 kg of anhydrous ethanol when the stirring paddle was at 400 rpm. After finishing adding the ethanol, the stirring was stopped, and alcohol precipitation was performed for 24 h; the supernatant was taken and concentrated under reduced pressure to obtain extractum of Ramulus Mori extract. Contents in sample: alkaloid had a content of 80%, polysaccharide had a content of 5%, flavone had a content of 0.1%, and amino acid had a content of 4%.

**Embodiment 5 Preparation 5 of mulberry extract**

400 kg of dried Ramulus Mori (Yuesang No. 11) was taken and crushed, then was added with 4000 L of water and was extracted twice by heating reflux, 1 h for each reflux; extraction solution was combined and filtered, and was concentrated until the crude drug quantity reached 1 kg/L.

62.5 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected extract was concentrated and loaded onto the anion resin, and the effluent was collected.

The collection solution obtained after anion column separation was filtered by means of micro-filtration membrane to remove impurities, then was concentrated through a reverse osmosis membrane, and was further concentrated under reduced pressure and dried to obtain extractum of Ramulus Mori extract. Contents in sample: alkaloid had a content of 3%, polysaccharide had a content of 70%, flavone had a content of 10%, and amino acid had a content of 10%.

**Embodiment 6 Preparation 6 of mulberry extract**

1500 kg of fresh Ramulus Mori (Morus serrata Roxb-Yuesang No. 11) was taken and crushed, then was added with 6000 L of water and was extracted by heating reflux for 2 h; extraction solution was combined, and was filtered to remove insoluble substances, and a crude extraction solution is thus obtained. The crude extraction solution was concentrated by heating until a percentage of solid substances was up to 4%, and was kept at 50°C and served as a loading solution for a cation resin column.

100 kg of D113-type macroporous and weakly acidic phenylpropene-based cation resin was used for column packing, and a hydrochloric acid solution of 2 mol/L was used for washing until the pH of an eluent was 4.5; a sodium hydroxide solution of 1 mol/L was used for washing until the pH of an eluent was 8.5; a hydrochloric acid solution of 2 mol/L was used for washing until the pH of an eluent was 4.5; and deionized water, of which the volume was 5 times as that of the column, was used for rinsing, and activation was thus completed. The concentrated extraction solution was loaded, and then was eluted with 1000 L of 2.5 mol/L ammonia water at an elution speed of 6 BV/h; the eluent was collected when the effluent from the cation column was detected to be pH>7; collection was stopped when the collection solution was up to 900 L, and the collection solution was purified directly through the anion column.

62.5 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected cation resin eluent was loaded onto the anion resin, and the effluent was collected until the effluent was up to 870 L. The effluent was concentrated under reduced pressure so as to obtain extractum of Ramulus Mori extract, wherein alkaloid had a content of 30%, polysaccharide had a content of 35%, flavone had a content of 2%, and amino acid had a content of 25%.

**Embodiment 7 Preparation 7 of mulberry extract**

1000 kg of fresh Ramulus Mori (Morus serrata Roxb-Yuesang No. 11) was taken and crushed, then was added with 4000 L of water and was extracted by heating reflux for 2 h; extraction solution was combined, and was filtered to remove insoluble substances, and a crude extraction solution is thus obtained. The crude extraction solution was concentrated by heating until a percentage of solid substances was up to 4%, and was kept at 50°C and served as a loading solution for a cation resin column.

100 kg of D113-type macroporous and weakly acidic phenylpropene-based cation resin was used for column packing, and a hydrochloric acid solution of 2 mol/L was used for washing until the pH of an eluent was 4.5; a sodium hydroxide solution of 1 mol/L was used for washing until the pH of an eluent was 8.5; a hydrochloric acid solution of 2 mol/L was used for washing until the pH of an eluent was 4.5; and deionized water, of which the volume was 5 times as that of the column, was used for rinsing, and activation was thus completed. The concentrated extraction solution was loaded, and then was eluted with 1000 L of 2.5 mol/L ammonia water at an elution speed of 6 BV/h; the eluent was collected when the effluent from the cation column was detected to be pH>7; collection was stopped when the collection solution was up to 900 L, and the collection solution was purified directly through the anion column.

62.5 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected cation resin eluent was loaded onto the anion resin, and the effluent was collected until the effluent was up to 870 L. The effluent was concentrated under reduced pressure so as to obtain extractum of Ramulus Mori extract, wherein alkaloid had a content of 40%, polysaccharide had a content of 25%, flavone had a content of 0.5%, and amino acid had a content of 25%.

**Embodiment 8 Preparation 8 of mulberry extract**

333 kg of dried Ramulus Mori (Yuesang No. 11) was taken and crushed, then was added with 4000 L of water and was extracted twice by heating reflux, 1 h for each reflux; extraction solution was combined and filtered, and was concentrated until the crude drug quantity reached 1 kg/L.

150 kg of D113-type macroporous and weakly acidic phenylpropene-based cation resin was used for column packing, and a hydrochloric acid solution of 2 mol/L was used for washing until the pH of an eluent was 4.5; a sodium hydroxide solution of 1 mol/L was used for washing until the pH of an eluent was 8.5; a hydrochloric acid solution of 2 mol/L was used for washing until the pH of an eluent was 4.5; and deionized water, of which the volume was 5 times as that of the column, was used for rinsing, and activation was thus completed. The concentrated extraction solution was loaded, and then was eluted with 1000 L of 2.5 mol/L ammonia water at an elution speed of 6 BV/h; the eluent was collected when the effluent from the cation column was detected to be pH>7; collection was stopped when the collection solution was up to 900 L, and the collection solution was purified directly through the anion column.

62.5 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected cation resin eluent was loaded onto the anion resin, and the effluent, of which pH was greater than 8, was collected until the effluent was up to 870 L.

The collection solution obtained after anion column separation was filtered by means of micro-filtration membrane to remove impurities, and then was concentrated through a reverse osmosis membrane, wherein the specific gravity of the concentrated liquid was 1.1; it was transferred to an alcohol precipitation tank, and was added with 15 kg of anhydrous ethanol when the stirring paddle was at 400 rpm. After finishing adding the ethanol, the stirring was stopped, and alcohol precipitation was performed for 24 h; the supernatant was taken and concentrated under reduced pressure to obtain extractum of Ramulus Mori extract. Contents in sample: alkaloid had a content of 63%, polysaccharide had a content of 23%, flavone had a content of 1%, and amino acid had a content of 5%.

**Embodiment 9 Effects of mulberry extract on the weight of KKAy mice with spontaneous type-2 diabetes**

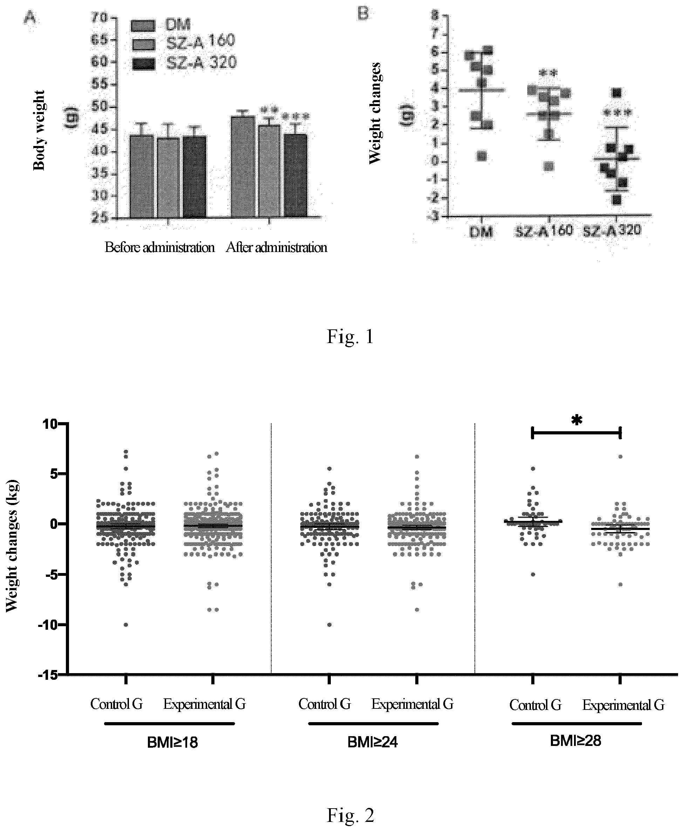

Twelve-week-old female KKAy mice were selected; after being fed with high-fat forage for three weeks, they were divided evenly into 3 groups (DM group, SZ-A 160 (mg mulberry extract/kg), SZ-A320 (mg mulberry extract/kg)) according to random blood glucose, fasting blood glucose, weight and other indicators, with 8 mice in each group, and were given mulberry extract of Embodiment 8 once a day by means of intragastric administration; the drug was administered continuously for about 6 weeks, and weight changes of the mice before and after administration were recorded, as shown in Table 1, Table 2 and Fig. 1.

Table 1 Grouped data of KKAy mice with spontaneous type 2 diabetes

| Group | Dose (mg extract/kg) | Fasting blood glucose (mg/dl) | Random blood glucose (mg/dl) | weight (g) |
|---|---|---|---|---|
| DM | - | 276.5±70.6 | 416.3±81.3 | 43.7±2.6 |
| SZ-A | 160 | 274.9±82.6 | 429.0±132.3 | 43.0±3.1 |
| | 320 | 274.6±66.0 | 420.2±140.7 | 43.4±2.1 |

Before the mulberry extract was given, the average body weight of mice in each group was about 43 g. About 6 weeks after administration of mulberry extract, the weight of mice in two dose groups of mulberry extract was significantly reduced, namely, the weight of mice in SZ-A160 dose group was lower than that in DM group by 2.0 g, the weight loss rate being 4.2%, and the weight of mice in SZ-A320 dose group was lower than that in DM group by 4.1 g, the weight loss rate being 8.6%. During administration, the weight gain rate of mice in the two SZ-A dose groups also decreased significantly, wherein: the average weight gain of mice in DM group was 4.4 g, the average weight gain of mice in SZ-A160 dose group was 2.6 g, and the average gain in SZ-A320 dose group was 0.1 g, indicating that mulberry extract can notably control the weight gain of KKAy mice.

Table 2 Weight changes of KKAy mice with spontaneous type 2 diabetes

| Group | Dose (mg extract/kg) | Weight before administration (g) | Weight after administration (g) | Weight gain (g) |
|---|---|---|---|---|
| DM | - | 43.7±2.6 | 47.6±1.4 | 4.4±1.5 |
| SZ-A | 160 | 43.0±3.1 | 45.6±1.6** | 2.6±1.3** |
| | 320 | 43.4±2.1 | 43.5±2.4*** | 0.1±1.6*** |

Relative to DM, data were expressed as X±SD, n=8; DM was the control group of KKAy model, SZ-A were drug administration groups. **P<0.01, ***P<0.001, relative to DM group

**Embodiment 10 Clinical experiment 1**

Ramulus Mori extract of Embodiment 1 was taken, and was added with an appropriate amount of auxiliary material; after even mixing and adding of water, a soft material was made, and then was granulated and dried; magnesium stearate was added and evenly mixed, and tablets were obtained via compression, thereby obtaining preparation of Ramulus Mori extract, each tablet containing 50 mg of total alkaloids.

Clinical cases and treatment:

(1) A 43-year-old Chinese male (84.2 kg, 170 cm, type 2 diabetes for 3 months) orally took tablets containing mulberry extract in Embodiment 1 (three times a day and two tablets each time orally for 24 weeks), and lost 10.2 kg in weight.
(2) A 65-year-old male (68.3 kg, 173 cm, type 2 diabetes for 7 months with hyperlipidemia) orally took tablets containing mulberry extract in Embodiment 1 (three times a day and two tablets each time continuously for 24 weeks), and lost 6.3 kg in weight.
(3) A 64-year-old male (75.0 kg, 168 cm, type 2 diabetes for 12 months) took acarbose for two months and then stopped; 8 months later, he orally took tablets containing mulberry extract in Embodiment 1 (three times a day and two tablets each time continuously for 24 weeks), and lost 7 kg in weight.
(4) A 48-year-old female (81.0 kg, 164 cm, type 2 diabetes for 8 months with hyperlipidemia) orally took tablets containing mulberry extract in Embodiment 1 (three times a day and two tablets each time continuously for 24 weeks), and lost 7 kg in weight.

**Embodiment 11 Clinical experiment 2**

1. Objective

Effect of Ramulus Mori extract on reducing weight of diabetics.

2. Experiment method

2.1 Grouping

Random, double-blind and double-simulation, parallel control of acarbose tablets, multicenter, non-inferiority were adopted in the study design. A total of 600 patients with type 2 diabetes (aged 18 to 70 years old, 19 kg/m$^2\leq$BMI$\leq$30 kg/m$^2$, 7%$\leq$HbA1C$\leq$10%) and poor blood glucose control were included, and were randomly divided into a total alkaloid tablet group (SZ-A, N=360) and a acarbose (Glucobay$^®$) group (CON, N=240). All signed informed consents.

The main exclusion criteria were as follows: 1) allergy or intolerance to $\alpha$-glucosidase inhibitors; 2) change of FBS levels between the first and second follow-up was greater than 2.5 mmol/L (>45 mg/dL); 3) history of severe diabetic complications (proliferative stage of diabetic retinopathy, diabetic nephropathy stage V, diabetic ketoacidosis, diabetic hypertonic coma, diabetic lactic acidosis); 4) drug combination therapy affecting glucose metabolism, such as anti-diabetic Chinese medicine or glucocorticoids; 5) hyperlipidemia accompanied by a history of irregular intake of lipid-lowering drugs; 6) chronic gastrointrialinal dysfunction, obvious digestive and absorption disorders or endocrine disorders such as hyperthyroidism, hypercortisolism and acromegaly; 7) severe heart disease, myocardial infarction, unstable angina pectoris, chronic cardiac insufficiency, or poor blood pressure control; 8) impaired liver or kidney function; and 9) pregnancy.

2.2 Administration method

Experimental groups orally took the tablets (tablets were prepared in the way below: extract of Embodiment 1 was taken, and was added with an appropriate amount of auxiliary materials; after even mixing and adding of water, a soft material was made, and then was granulated and dried; magnesium stearate was added and evenly mixed, and tablets were obtained via compression, thereby obtaining preparation of Ramulus Mori extract, each tablet containing 50 mg of total alkaloids) with an initial dose of one tablet for each time and three times a day; four weeks later, the dose successively increased to two tablets for each time and three times a day; the control group orally took acarbose, one tablet each time and three times a day.

3. Detection and processing of experiment data

3.1 Detection index

(1) From the 1$^{st}$ to the 24$^{th}$ week of administration, the height, weight and weight gain of each group were observed and recorded every day.

3.2 Statistical analysis

SPSS 20.0 software was used for data processing, and differences between groups were tested by T test.

4. Results

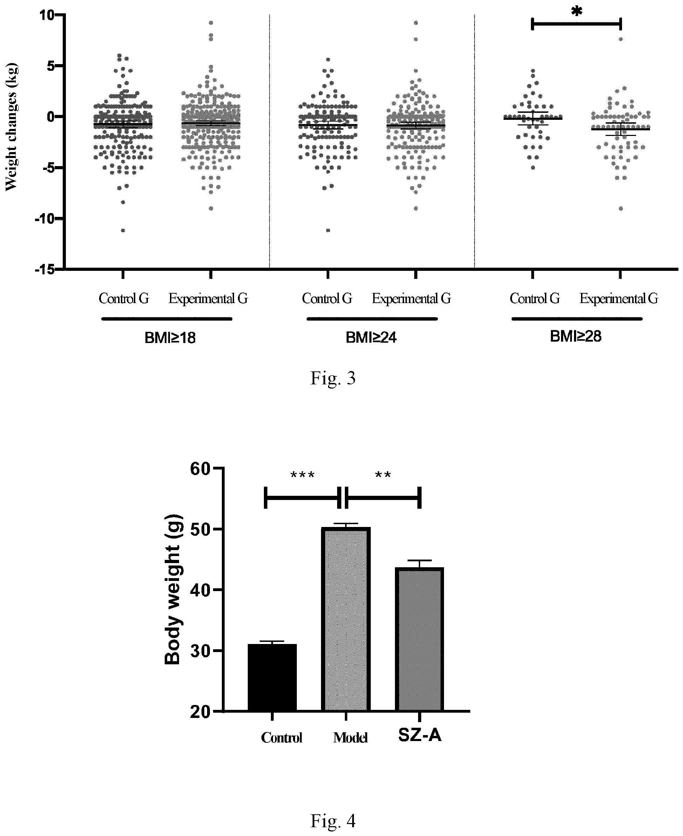

After 8 weeks and 24 weeks of administration, the experiment data of different BMI groups of type 2 diabetics in each group were obtained, as shown in Tables 3 and 4, and Figs. 2 and 3.
After 8 weeks of administration, as for patients with BMI$\geq$28 kg/m$^2$, weight in the experimental group was obviously reduced, compared with that in the control group, by an average change of -0.503 kg and 0.210 kg respectively (P=0.016); after 24 weeks of administration, as for patients with BMI$\geq$28 kg/m$^2$, weight in the experimental group was obviously reduced, compared with that in the control group, by an average change of -1.228 kg and -0.182 kg respectively (P=0.022); for other BMI groups, weight changes were not statistically significant.

Table 3: Test analysis of independent samples after 8 weeks

|  | BMI$\geq$18 kg/m$^2$ | BMI$\geq$24 kg/m$^2$ | BMI$\geq$28 kg/m$^2$ |
|---|---|---|---|
| P | 0.719 | 0.714 | 0.016 |

Table 4: Test analysis of independent samples after 24 weeks

|  | BMI$\geq$18 kg/m$^2$ | BMI$\geq$24 kg/m$^2$ | BMI$\geq$28 kg/m$^2$ |
|---|---|---|---|
| P | 0.664 | 0.84 | 0.022 |

It is shown that the use of Ramulus Mori extract in diabetics with BMI$\geq$28 kg/m$^2$ could treat type 2 diabetes, as well as reduce weight.

**Embodiment 12 Clinical experimental example 3**

1. Objective

Effect of Ramulus Mori extract on reducing weight of healthy people.

2. Experiment method

2.1 Experiment instruments and materials

Weight scale.

2.2 Experiment design

A total of 17 cases in a non-diabetic group, including 11 males and 6 females, were all healthy people; they were on regular diets during the medication period; the medication period was 0.7 to 8 months, the dose was 50 or 100 mg/time, and the frequency of medication was 2 to 3 times/day.

2.3 Detection index

Before and at the end of the experiments, the following body indicators were detected: weight, height.
Judgement criteria of weight loss: (1) excellent, wherein the weight loss percentage is greater than or equal to 10%; (2) obvious, wherein the weight loss percentage is greater than or equal to 5% and less than 10%; (3) effective, wherein the percentage of weight loss is greater than or equal to 3% and less than 5%; and (4) ineffective, wherein the weight loss percentage is less than 3%.

3. Results

There were 2 cases of excellent, 9 cases of obvious, 5 cases of effective and 1 case of ineffective, and the total effective rate was 94%. In addition, there were no adverse reactions after taking of the drug in all subjects, indicating that the drug has a significant effect on weight loss in non-diabetic patients. Information and experimental results of the subjects are shown in Table 5 below.

Total effective rate= (Excellent case + Obvious cases + Effective cases)/Cases*100%.

TID: the drug is taken three times a day

BID: the drug is taken twice a day

Table 5

| No. | Gender | Age | Height (m) | Medication period (month) | Dose (mg/ time) | Usage | Weight before medication kg | Weight after medication kg | Percentage of weight loss (%) |
|---|---|---|---|---|---|---|---|---|---|
| 1 | Male | 50 | 1.70 | 6 | 50 | TID | 70 | 61 | 13 |
| 2 | Male | 45 | 1.72 | 3 | 100 | TID | 68 | 64 | 6 |
| 3 | Male | 42 | 1.74 | 3 | 50 | BID | 92 | 84 | 9 |
| 4 | Male | 43 | 1.71 | 8 | 100 | TID | 76 | 69.5 | 9 |
| 5 | Male | 33 | 1.9 | 1 | 50 | BID | 103.4 | 98.7 | 5 |
| 6 | Female | 43 | 1.59 | 6 | 50 | TID | 49 | 46 | 6 |
| 7 | Female | 30 | 1.68 | 1 | 50 | TID | 66 | 62 | 6 |
| 8 | Male | 58 | 1.66 | 6 | 50 | TID | 68 | 65 | 4 |
| 9 | Female | 56 | 1.58 | 2 | 50 | TID | 58 | 56 | 3 |
| 10 | Male | 53 | 1.76 | 2 | 50 | BID | 90 | 88.5 | 2 |
| 11 | Female | 40 | 1.6 | 2 | 50 | BID | 65 | 62 | 5 |
| 12 | Male | 46 | 1.75 | 1 | 50 | TID | 77.5 | 75 | 3 |
| 13 | Male | 34 | 1.72 | 4 | 50 | BID | 80 | 73 | 9 |
| 14 | Male | 35 | 1.7 | 3 | 100 | BID | 77 | 72 | 6 |
| 15 | Female | 46 | 1.57 | 1.5 | 100 | TID | 62 | 59.5 | 4 |
| 16 | Male | 28 | 1.87 | 1 | 50 | TID | 110 | 105.3 | 4 |
| 17 | Female | 49 | 1.6 | 0.7 | 100 | TID | 65 | 56 | 14 |

**Embodiment 13 Weight loss experiment of high-fat fed C57 mice**

1. Intragastric administration

Thirty healthy 6-week-old male C57 mice were randomly divided into a normal group, a model group and a SZ-A group, with 10 mice in each group, wherein: mice in the normal group were fed with basic food, and mice in the model group and SZ-A group were on a high-fat diet. After 14 weeks of feeding, mice in each group were given corresponding drug by means of intragastric administration every day for 6 consecutive weeks, wherein: the SZ-A group was given intragastric administration of 400 mg/kg/d according to total alkaloids of Ramulus Mori, and the normal group and the model group were given intragastric administration of corresponding dose of solvent. During drug treatment, the weight of mice was monitored. After finishing drug administration, all mice were weighed after fasting of 12 hours.

As shown in Fig. 4, the average weight of mice in the model group was 50.3 g, and that of mice in the administration group was 43.7 g, indicating that SZ-A could significantly reduce the weight of mice on a high-fat diet.

2. Injection administration

(1) Animal modeling: thirty 6-week-old C57 mice were randomly divided into three groups, wherein: the first group was fed with basic food, the second and third groups were on a high-fat diet, and the feeding lasted for 14 weeks.
(2) Administration: based on weight, the third group was given intraperitoneal injection of 200 mg/kg of Ramulus Mori total alkaloids, and the injection administration period was 6 weeks. The first and second groups were individually injected once a day with normal saline according to weight.
(3) Weight measurement: after administration, the weight of mice was measured with a balance and was recorded.

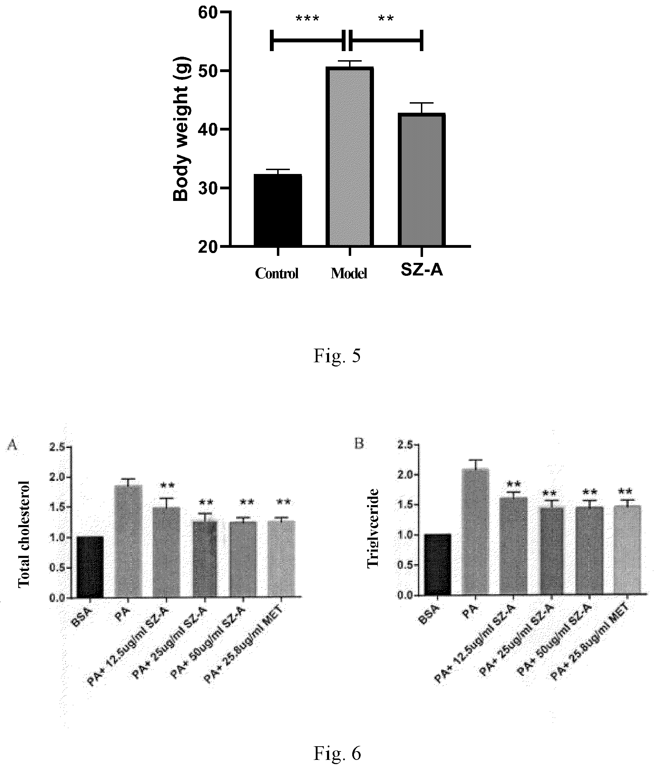

As shown in Fig. 5, the average weight of the model group was 50.6 g, and that of the administration group was 42.8 g, indicating that the injection of SZ-A could significantly reduce obesity caused by high-fat diet.

**Embodiment 14 In vitro cell experiment**

1. After digestion of HepG2 cells in logarithmic growth stage, the cell density was adjusted to $2 \times 10^5$ cells/ml with DMEM (high glucose) complete culture medium containing 10% FBS, and cells was transferred into a 6-well plate by 2 ml/well.
2. After culturing of 24 h, model cells were randomly divided into a SZ-A group (Ramulus Mori extract SZ-A in Embodiment 1 at 50 ug/ml, 25 ug/ml and 12.5 ug/ml), a metformin group (200 umol/l of MET) and a palmitic acid stimulated model group (PA); meanwhile, HepG2 cells served as a normal control group (0.25% BSA). In addition to the normal control group (0.25% BSA) and the model group (PA), corresponding drugs and palmitic acid were individually added to other groups, the palmitic acid stimulation model was replicated, and three multiple holes were provided for each group.
3. After culturing of 24 h, preheated PBS was added for washing once, and then each well of the 6-well plate was added with 400 ul of Triton x-100 lysate containing 1% PMSF. The 6-well plate was cracked on ice or at 4°C for 1 h with a pipette gently blowing and beating so that the lysate and cells were in full contact. The lysed liquid was not centrifuged, and 10 ul of triglyceride and 10 ul of total cholesterol were directly measured after swirling.

As shown in Fig. 6, excessive accumulation of fat in liver could easily cause fatty liver. Fatty liver formation can be simulated by stimulating HepG2 liver cells with palmitic acid PA. When different doses of SZ-A were added, accumulation of triglycerides and cholesterol in liver caused by inhibition of palmitic acid by means of SZ-A could be seen. The effect of SZ-A is similar to that of MET.

With reference to the afore-mentioned method, the model cells were treated with the mulberry extract (the dose was 25 ug/ml) prepared in Embodiments 2 to 7, and the triglycerides and total cholesterol in cells were respectively measured. According to the results, relative to the PA group, the accumulation of triglycerides and cholesterol in liver caused by palmitic acid was decreased to a certain extent after treatment by means of mulberry extract. Specific results are shown in Table 6 below.

Table 6 Effects of mulberry extract prepared in Embodiments 1 to 7 on triglyceride and cholesterol levels

| Mulberry extract | Triglyceride level (Treatment group/PA group) | Cholesterol level (Treatment group/PA group) |
|---|---|---|
| Ramulus Mori extract in Example 1 | 55% | 55% |
| Ramulus Mori extract in Example 2 | 30% | 40% |
| Ramulus Mori extract in Example 3 | 80% | 75% |
| Ramulus Mori extract in Example 4 | 40% | 40% |
| Ramulus Mori extract in Example 5 | 90% | 90% |
| Ramulus Mori extract in Example 6 | 60% | 65% |
| Ramulus Mori extract in Example 7 | 70% | 70% |

The above embodiments show that mulberry extract plays a good role in weight controlling. Long-term administration of mulberry extract can inhibit the weight growth of mice and human beings, and inhibit the weight gain of type 2 diabetic mice and diabetic patients; besides, it can inhibit the accumulation of fat in organs.

With reference to preferable embodiments, the present disclosure is explained in the above way. However, these embodiments are merely exemplary and illustrative. On such a basis, a variety of replacements and improvements can be made to the present disclosure, and all these replacements and improvements fall within the scope of protection of the present disclosure.

## Claims

1. Use of a mulberry extract in preparation of a drug for reducing animal weight, wherein: the mulberry extract comprises an alkaloid, a polysaccharide, an amino acid and flavone, and, on the basis of the mulberry extract, weight contents of each component are as follows:

   | an alkaloid | 3% to 99%, |
   |---|---|
   | a polysaccharide | 0.2% to 70%, |
   | flavone | 0% to 10%, |
   | an amino acid | 0% to 50%, and |
   | other components | 0% to 25%; |

   more preferably, on the basis of the mulberry extract, weight contents of each component are as follows:

   | an alkaloid | 50% to 99%, |
   |---|---|
   | a polysaccharide | 0.2% to 35%, |
   | flavone | 0% to 2%, |
   | an amino acid | 0% to 30%, and |
   | other components | 0% to 20%. |

2. The use according to claim 1, wherein the animals are mammals, including humans and rodents.

3. The use according to claim 1 or 2, wherein the animals are diabetic.

4. The use according to claim 1 or 2, wherein the animals are healthy.

5. The use according to claim 1, wherein the drug further comprises a pharmaceutically acceptable carrier.

6. The use according to claim 1, wherein the drug has a dosage form of oral administration; preferably, the drug is a tablet, a capsule, an oral solution, an oral emulsion, a pill, a granule, syrup and powder.

7. The use according to claim 3, wherein the diabetic animals have a BMI$\geq$28.

Fig. 1

Fig. 2

Fig. 3

Fig. 4

Fig. 5

Fig. 6

## INTERNATIONAL SEARCH REPORT

International application No.

**PCT/CN2022/080278**

### A. CLASSIFICATION OF SUBJECT MATTER

A61K 36/605(2006.01)i;  A61P 3/04(2006.01)i;  A61P 3/06(2006.01)i;  A61P 3/10(2006.01)i

According to International Patent Classification (IPC) or to both national classification and IPC

### B. FIELDS SEARCHED

Minimum documentation searched (classification system followed by classification symbols)

A61K,  A61P

Documentation searched other than minimum documentation to the extent that such documents are included in the fields searched

Electronic data base consulted during the international search (name of data base and, where practicable, search terms used)

CNABS, DWPI, VEN, CNTXT, CNMED, CNKI, USTXT, EPTXT, WOTXT, SIPOABS, web of science, 中国药物专利数据库 (网页), Chinese Pharmaceutical Patent Database (webpage), 药品标准数据库 (E药全库和国家新药注册数据库), Drug Standard Database (E-medicine Full-Text Database and National New Drug Registration Database), 超星, CHAOXING, 读秀, DUXIU, 百度, BAIDU: 桑, 桑枝, 桑白皮, 桑叶, 桑椹, 减重, 减肥, 体重, 肥胖症, 糖尿病, morusalba, ramulus mori, cortex mori, folium mori, mulberry, lose weight, weight reduce, weight, obesity, diabetes

### C. DOCUMENTS CONSIDERED TO BE RELEVANT

| Category* | Citation of document, with indication, where appropriate, of the relevant passages | Relevant to claim No. |
|---|---|---|
| PX | CN 113143997 A (BEIJING WEHAND PHARMACEUTICAL CO., LTD. et al.) 23 July 2021 (2021-07-23)<br>claims 1-5 | 1-7 |
| X | CN 111658692 A (BEIJING WEHAND BIO PHARMACEUTICAL CO., LTD. et al.) 15 September 2020 (2020-09-15)<br>claims 1-2, 4, 6, and 8-9, and description, paragraphs 0057-0060 | 1-7 |
| A | CN 111568948 A (BEIJING WEHAND BIO PHARMACEUTICAL CO., LTD. et al.) 25 August 2020 (2020-08-25)<br>entire document | 1-7 |
| A | CN 1631246 A (SERICULTURE & AGRI-FOOD RESEARCH INSTITUTE GAAS et al.) 29 June 2005 (2005-06-29)<br>entire document | 1-7 |

☑ Further documents are listed in the continuation of Box C.   ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **19 May 2022** | **30 May 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/080278**

**C.   DOCUMENTS CONSIDERED TO BE RELEVANT**

| Category* | Citation of document, with indication, where appropriate, of the relevant passages | Relevant to claim No. |
|---|---|---|
| A | 刘玉玲等 (LIU, Yuling et al.). "桑枝总生物碱研发历程回顾(一)：药学研究技术壁垒与规模化发展的挑战 (Review of the research and development of Ramulus Mori(Sangzhi) alkaloids(I) : technical barriers and large-scale development challenges in pharmaceutical research)"<br>中国糖尿病杂志 (Chinese Journal of Diabetes), Vol. 28, No. 7, 31 July 2020 (2020-07-31),<br>pp. 555-560 | 1-7 |
| A | 刘率男等 (LIU, Shuainan et al.). "桑枝总生物碱研发历程回顾(二): 现代药理学理念诠释中药的药效特点及药理作用机制 (Review of the research and development of Ramulus Mori(Sangzhi) alkaloids(II): modern pharmacological concepts interpret the characteristics of pharmacological effects and mechanisms of traditional Chinese medicines)"<br>中国糖尿病杂志 (Chinese Journal of Diabetes),<br>Vol. 28, No. 8, 31 August 2020 (2020-08-31),<br>pp. 635-640 | 1-7 |

Form PCT/ISA/210 (second sheet) (January 2015)

**INTERNATIONAL SEARCH REPORT**
Information on patent family members

International application No.

**PCT/CN2022/080278**

| Patent document cited in search report | | | Publication date (day/month/year) | Patent family member(s) | Publication date (day/month/year) |
|---|---|---|---|---|---|
| CN | 113143997 | A | 23 July 2021 | None | |
| CN | 111658692 | A | 15 September 2020 | None | |
| CN | 111568948 | A | 25 August 2020 | None | |
| CN | 1631246 | A | 29 June 2005 | None | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 1631246 A
- CN 102370708 A
- CN 111077247 A
- CN 110393738 A

**Non-patent literature cited in the description**

- Experts' Consensus in China on Integrated Management of Type 2 Diabetes with Obesity. *China J Endocrinol Metab,* 2016, vol. 32 (08), 623-627